Europäisches Patentamt

European Patent Office

Office européen des brevets

(11) Publication number: **0 319 873**
**A2**

## EUROPEAN PATENT APPLICATION

(21) Application number: 88120213.9

(22) Date of filing: 03.12.88

(51) Int. Cl.4: **G01N 33/49 , G01N 27/28**

(30) Priority: 09.12.87 US 130569

(43) Date of publication of application:
14.06.89 Bulletin 89/24

(84) Designated Contracting States:
BE CH DE ES FR GB GR IT LI NL SE

(71) Applicant: **INTERNATIONAL CARDIOVASCULAR MEDICINE, INC.**
**18345 Ventura Boulevard Suite 211**
**Tarzana California 91356(US)**

(72) Inventor: **Burnam, Michael H.**
**23472 Rolling View Drive**
**Hidden Hills California 91302(US)**

(74) Representative: **Hoormann, Walter, Dr.-Ing. et al**
**FORRESTER & BOEHMERT**
**Widenmayerstrasse 4/I**
**D-8000 München 22(DE)**

(54) Method and apparatus for single determination blood analysis.

(57) The present invention relates to a method and apparatus for analyzing blood using a disposable sensing unit which is configured for single determination analysis of certain constituents of blood.

Fig.3

## METHOD AND APPARATUS FOR SINGLE DETERMINATION BLOOD ANALYSIS.

### BACKGROUND OF THE INVENTION

#### 1. Field of the Invention

The present invention relates to a new method and improved apparatus for analyzing blood for selected components by means of a disposable electrode sensing unit which is configured for single determination analysis of ion components of blood. In an embodiment, the present invention provides a method and apparatus for analyzing a relatively small amount of blood using a relatively small self-contained disposable electrode for single determination analysis of ion components of blood.

#### 2. Description of the Prior Art

There are generally two ways of analyzing a liquid for its various constituents. The first of these is a noncontinuous sampling method in which a volume of the liquid is taken and is then analyzed for the various constituents under consideration.

The second method is a continuous analysis method in which all or part of the liquid is allowed to pass through an analytical apparatus.

An advantage of the first method is its accuracy. However, two serious disadvantages of prior art devices have been the time taken for the test and the relatively large quantity of the liquid that must be removed which cannot thereafter be replaced. An advantage of the second method is that the liquid which is used for sampling is flowing continuously and therefore takes into account variations in the level of constituents as they occur. Also, the liquid after analysis is usually returned to the bulk of the liquid. One problem is that the analytical apparatus contacts the liquid and, particularly if the liquid is blood, there is a risk of contamination of ion-selective electrodes in the apparatus by the blood.

Various clinical procedures have evolved over the years for determining alkali metals, notably sodium and potassium, in liquids and particularly in biological fluids such as blood serum. Such procedures are frequently used in monitoring renal functions, as well as being used to detect indications of other diseases. For example, in healthy human beings, the potassium level in the blood serum falls within a narrow range, generally between 3 and 6 millimoles per liter of blood serum. A potassium concentration of only 1.5 millimoles per liter can be fatal, resulting in respiratory depression and cardiac arrhythmia. Because of the range of normal potassium level is limited and further because a relatively small deviation from the normal range can be devastating, clinical procedures for determining alkali metal levels must be highly accurate.

Measurement of the potassium level in blood aids the physician in diagnosis of conditions leading to muscle irritability and excitatory changes in myocardial function and conditions such as oliguria, anuria, urinary obstruction and renal failure due to shock. The measurement of potassium in serum is particularly important clinically. The measurement require high sensitivity and precision since the normal clinical range is only from about 2 to about 10 millimolar (mM) with a normal range from about 3.5 to 5.5 mM. Measurement of lithium levels in the blood is also important since the toxic dose levels are only slightly higher than the therapeutic levels used in psychiatric treatment.

Determination of sodium and potassium has long occupied a place near the top of the frequency list of required tests in clinical laboratories. A third blood metal, calcium, also important, has not been found as high in the list primarily because of its considerably greater difficulty of determination. The preferred method of determination of sodium and potassium for reasons of speed, ease and accuracy is by means of the flame photometer. In this instrument, after diluting, the sample is excited to optical emission in a flame and the intensity of this emission is read photometrically as a measure of the concentration of sodium and potassium. Calcium is generally determined by the Clark-Colip method, by fluorescence or by atomic absorption. The advent of ion-selective electrodes to date has not solved this dilemma either. Although the sodium electrode is reasonably fast the calcium electrode is slow and the potassium electrode lacks specificity.

In flame photometry conventionally serum or plasma is diluted by a predetermined amount by adding a suitable diluent. This diluent may contain constituents added to minimize instability of readings due to fluctuations in sample delivery to the flame. Constituents may also be added to compensate for or reduce optical or chemical interferences which would affect the emission/concentration relationships adversely. The greater the dilution factor the less will be the necessary volume of the original serum. However, the less also will be the emission intensity and the greater the demands on the photometric system.

Conventional methods for determining the alkali metal content, and specifically sodium and potassium contents, of fluids such as blood serum have been used clinically. Typical of such techniques are flame photometry and ion-specific potentiometry.

One improvement in techniques for determining alkali metals has been described in Sumiyoshi and Nakahara in Talanta, 24 (1977), 763. In the technique described, potassium was determined photometrically by first forming a complex between potassium and a monocyclic crown ether. The complex was then extracted into benzene as an ion pair with an anionic dye. Thus, the color intensity of the dye was a relatively accurate measure of the potassium content of the solution. Various other similar colorimetric methods have also been described in the literature.

One of the shortcomings colorimetric determinations of the prior art, including, is that all known methodologies require deproteinization of the serum or plasma being assayed prior to formation of the alkali metal-cryptand complex. The necessity for treatment to remove proteins can affect the alkali metal content of the serum or plasma and thus inroduce inaccuracies to the procedure.

It is readily apparent that a sensitive, convenient and inexpensive method for determining the ion concentration in blood would greatly enhance the state of these technologies, as well as any others where such rapid, accurate determinations would be beneficial. Thus, a medical laboratory technician could accurately measure the sodium, lithium, potassium, magnesium or calcium level of a serum or whole blood sample in a matter of seconds or minutes, such rapid results would increase laboratory efficiency and aid the physician in diagnosis.

## SUMMARY OF THE INVENTION

Accordingly, it is an object of the present invention to provide an improved method for the determination of ions in liquids, particularly in fluids such as blood serum or plasma, and which does not require prior deproteinization of such fluids.

It is another object of the invention to provide a method for the determination of selected ions in a simple, fast and specific to use determination.

It is yet another object of the invention to provide an apparatus for the determination of ions in blood in a simple, efficient, highly reliable manner using a one-determination disposable electrode.

It is a further object of this invention to provide a method and apparatus for selected ion determining using a disposable electrode which effects analysis on relatively small amounts of blood in a simple, safe and convenient manner.

It is yet a further object of this invention to provide a new and improved method and apparatus for selected ion determination by a patient using drop sizes of blood using an economically disposable electrode.

These and other objects and advantages will be readily apparent from the following detailed description of the invention taken in conjunction with the figures wherein similar elements are identified by like numerials throughout the several views.

## BRIEF DESCRIPTION OF THE DRAWINGS

Figure 1 is a front elevation of the apparatus.
Figure 2 is a front elevation view showing the removable electrode.
Figure 3 is a side view of the apparatus.

## DETAILED DESCRIPTION OF THE INVENTION

When two solutions having different concentrations of ions are separated by an electrically conductive membrane, an electrical potential (EMF) is generated. In membrane separation cells, the membrane can be a simple fritted glass barrier, allowing a small but measurable degree on ion diffusion from one solution to the other. Alternatively, a nonporous, electrically nonconductive film, such as polyvinyl chloride, impregnated with an ionophore can be employed. In the absence of the ionophore, the film is an insulator and no EMF can be measured; when blended with an ionophore, charged ions are bound to the film and a small, measurable current can be induced to flow. Because the ionophore is selective in its affinity, and thus will bind only certain specific ions, such cells are ion selective. Any measurable EMF is due solely to the presence of the bound ions.

A major difficulty in the use of such ion-selective electrodes has been the marked reduction of accuracy and speed of response over time. Further, small changes in ion concentration produce such small changes in EMF that sophisticated voltmeter equipment is required.

The apparatus to be described is for the direct analysis of patients blood and the concentration of certain constituents of clinical importance. The apparatus is designed for the measurement of potassium, however, it can be modified for use in the measurement of other electrolytes such as calcium, for example.

The apparatus may be calibrated by passing control liquid in place of the blood through the apparatus, having known proportions of constituents and mating the output readings.

## DETAILED DESCRIPTION OF THE DRAWINGS

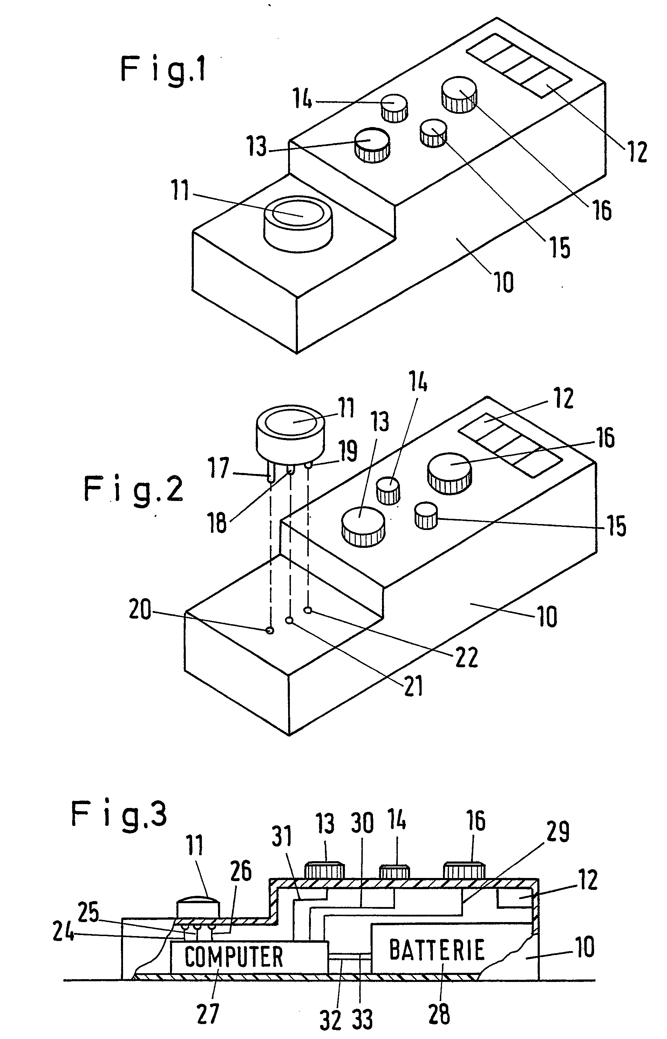

Referring now to Figure 1 which shows the case 10 having a detecting cell 11 positioned thereon and an element 12 which is a glass covering a reading device that records the electromotive force generated by the potassium that is contained in the blood as measured by the detecting cell 11.

Referring now to Figure 2 which shows the disposable detecting cell 11 in the elevated position after measurment of potassium content of the blood. The detecting cell 11 has a series of three electrodes 17, 18 and 19 that fit into the matching apertures 20, 21 and 22.

Referring now to Figure 3 which is a cross-sectional view of the apparatus shown in Figure 1. The view shows the computer 27 connected by means of connecting wires 24, 25 and 26 to the detecting cell 11. The apparatus is powered by a battery 28 that is connected to the knobs 13, 14 and 15 by means of wires 29, 30 and 31 into the computer by means of wires 32 and 33.

The essential feature of the device resides in the use of a small disposable detecting cell. The cell is coated with a non-porous electrically conducting film of polyvinyl chloride. The film is impregnated with an ionophore that is specific to the detection of potassium. The ionophore can be modified for the detection of other ions such as calcium, for example.

In use, the technician removes the disposable pre-calibrated detecting cell from a sanitized package and positions it in the instrument. A drop of blood is positioned on the detecting cell and the electromotive force generated is shown on the reading device that is calibrated to the concentration of potassium or other ions.

When the test is completed the detecting cell is discarded and a new cell is used for any subsequent test.

Obviously, many modifications and variations in the invention may be made without departing from the essence and scope thereof, and only such limitations should be applied as are indicated in the appended claims.

## Claims

1. An apparatus for analyzing blood using a disposable detecting cell designed for a single determination analysis of certain constituents of blood which compresses in combination,

(a) a case containing a battery and means for computing electromotive force, operably connected to a readout device,

(b) a disposable detecting cell coated with a non-conductive film impregnated with an ionophore specific for the ion to be measured,

(c) calibrated readout means for displaying the electromotive force generated by the ion added to the detecting cell.

2. The apparatus according to claim 1 wherein said detecting cell is coated with polyvinyl chloride impregnated with an ionophore specific for potassium.

3. The apparatus according to claim 1 wherein the readout device displays the potassium content in milimoles per liter.

EP 0 319 873 A2

Fig.1

Fig.2

Fig.3